# Europäisches Patentamt
# European Patent Office
# Office européen des brevets

(19)

(11) Veröffentlichungsnummer: **0 318 989 B1**

(12) **EUROPÄISCHE PATENTSCHRIFT**

(45) Veröffentlichungstag der Patentschrift: **08.03.95**

(51) Int. Cl.6: **C08J 3/12**, A61L 15/00

(21) Anmeldenummer: **88120065.3**

(22) Anmeldetag: **01.12.88**

Verbunden mit 88910033.5/0394298 (europäische Anmeldenummer/Veröffentlichungsnummer) durch Entscheidung vom 21.10.91.

(54) **Polymerisate mit hoher Aufnahmegeschwindigkeit für wässrige Flüssigkeiten.**

(30) Priorität: **04.12.87 DE 3741158**

(43) Veröffentlichungstag der Anmeldung:
**07.06.89 Patentblatt 89/23**

(45) Bekanntmachung des Hinweises auf die Patenterteilung:
**08.03.95 Patentblatt 95/10**

(84) Benannte Vertragsstaaten:
**AT BE CH DE ES FR GB GR IT LI LU NL SE**

(56) Entgegenhaltungen:
**EP-A- 0 001 706**
**GB-A- 1 376 091**
**GB-A- 2 040 954**

(73) Patentinhaber: **Chemische Fabrik Stockhausen GmbH**
**Bäkerpfad 25**
**D-47805 Krefeld (DE)**

(72) Erfinder: **Chmelir, Miroslav, Dr.**
**Grönkesdyk 35**
**D-4150 Krefeld (DE)**
Erfinder: **Stukenbrock, Karl-Heinz**
**Panoramaweg 25**
**D-4054 Nettetal (DE)**

(74) Vertreter: **Klöpsch, Gerald, Dr.-Ing. Patentanwalt**
**An Gross St. Martin 6**
**D-50667 Köln (DE)**

**Beschreibung**

Die Erfindung betrifft Polymerisate, die Wasser und wäßrige Flüssigkeiten schnell aufnehmen, die z.B. in absorbierenden Wegwerferzeugnissen für Hygieneartikel (Windeln und Damenbinden) und für andere medizinische Zwecke, oder auch als wasserspeichernde Bodenverbesserungsmittel eine Verwendung finden, sowie ein Verfahren zu ihrer Herstellung und ihre Verwendung als Absorptionsmittel für Wasser und wäßrige Flüssigkeiten.

In den letzten Jahren wurde eine Anzahl verschiedener Polymerisate entwickelt, die hohes Absorptionsvermögen für Wasser und Körperflüssigkeiten aufweisen. Die meisten Produkte wurden auf Stärkebasis, wie z.B. Stärke-Acrylnitril-Propfpolymerisate (US-PS 3,997,484; 3,661,815; 4,155,888; 3,935,099), gelatinisierte Stärkederivate (DE-OS 27 02 781) oder auf Cellulosebasis, wie Derivate von Alkyl- oder Hydroxy-alkylcellulose (JP-PS 77/125.481), Carboxymethylcellulose (BE-PS 862,130; GB-PS 1,159,949) und auf Polysaccharidbasis (DE-OS 26 50 377) hergestellt. Zu den vollsynthetischen, in zahlreichen Patenten beschriebenen Absorptionsmitteln gehören vernetzte Polymere und Copolymere auf Acryl- oder Methacrylsäurebasis (DE-OS 24 29 236; DE-OS 26 14 662; US-PS 4,018,951; US-PS 3,926,891; US-PS 4,066,583; US-PS 4,062,817; DE-OS 27 12 043; DE-OS 26 53 135; DE-OS 26 50 377; DE-OS 28 13 634), Maleinsäurederivate (US-PS 4,041,228) oder Acrylamidopropansulfonsäurecopolymerisate (DE-PS 31 24 008).

Die bekannten Absorptionsmittel dieser Art sind praktisch wasserunlöslich, absorbieren im Gleichgewicht das Vielfache ihres Gewichts an Wasser, Urin oder anderen wäßrigen Lösungen, wobei die relativ niedrige Aufnahmegeschwindigkeit der wäßrigen Flüssigkeiten bisher nur eine untergeordnete Rolle spielte, und sie wurde nur in wenigen Patentschriften überhaupt erwähnt. So wird z.B. in der DE-OS 28 13 634 ein Acrylsäure/Acrylnitril/Stearylmethacrylat-Mischpolymerisat beschrieben, das innerhalb von 30 Sekunden das 38-fache seines Eingengewichts an künstlichem Urin aufnimmt.

Gemäß DE-PS 31 28 100 kann die Beschleunigung der Flüssigkeitsaufnahme durch Zusatz von verschiedene n wasserlöslichen Substanzen wie Zuckern, Harnstoff oder Aluminiumsulfat erreicht werden.

Eine teilweise Verbesserung der Blutaufnahmegeschwindigkeit wurde nach den DE-OS 28 44 956 und EP-PS 0009977 dadurch erreicht, daß ein teilsynthetisches oder vollsynthetisches Absorptionsmitel in Pulverform nachträglich mit Polyethern (DE-OS 28 44 956) oder mit Fettalkoholen, Fettsäuren oder -estern (EP-PS 0 009 977), meistens gelöst in organischen Lösungsmitteln, behandelt wird.

Aufgabe der Erfindung ist es, die bekannten als Absorptionsmittel eingesetzten Polymerisate hinsichtlich ihrer Aufnahmegeschwindigkeit für Wasser und wäßrige Flüssigkeiten wie Urin oder Blut zu verbessern.

Diese Aufgabe wird gelöst durch wasserquellbare, natürliche oder synthetische Polymerisate mit hohem Absorptionsvermögen für Wasser und wässrige Flüssigkeiten, die durch Agglomerierung eines feinteiligen, pulverförmigen Ausgangsmaterials einer Korngröße von kleiner 0,2 mm, bevorzugt kleiner 0,1 mm, und insbesondere kleiner 0,05 mm mittels einer Lösung oder Dispersion eines Agglomerierungsmittels in Wasser, einem in Wasser mischbaren oder nicht mischbaren organischen Lösungsmittel oder einem Gemisch aus Wasser und einem solchen organischen Lösungsmittel und gegebenenfalls weiteren, noch agglomeriered wirkenden Zusätzen, erhältlich sind.

Durch die Agglomerierung, welche nach üblichen Verfahren durchgeführt wird, wird ein gröberes Korn als es das ursprüngliche Produkt hatte, erzielt, wobei bei dem agglomerierten Polymerisat eine Kornverteilung von 0,1 bis 0,5 und besonders von 0,2 bis 1,0 mm bevorzugt wird. Es ist aber auch möglich, Endprodukte mit Teilchen, die größer als 1 mm sind, herzustellen.

Die Agglomerierung wird bevorzugt mit einer wässrigen Lösung oder einem wasserhaltigen Gemisch des Agglomerierungshilfsmittels durchgeführt, indem das Agglomerierungshilfsmittel auf geeignete Weise feinverteilt mit dem zu agglomerierenden Polymerisat zusammengebracht wird. Bevorzugt wird das Agglomerierungshilfsmittel in wässriger Lösung oder Dispersion auf das zu agglomerierende Polymerisat aufgesprüht, während dieses in Bewegung gehalten wird. Das Agglomerierungshilfsmittel kann aber auch in einem mit Wasser mischbaren oder nicht mischbaren organischen Lösungsmittel oder einem Gemisch aus Wasser und einem solchen Lösungsmittel gelöst oder dispergiert werden. Die Agglomerierung erfolgt in der Wirbelschicht, die mittels eines geeigneten Inertgases, vorzugsweise Luft, betrieben werden kann. Die Luft wird dabei vorteilhaft gleich erwärmt, um das Wasser aus dem System zu entfernen und das Agglomerat zu trocknen. Apparativ hat sich für die Durchführung des Verfahrens ein Wirbelschichttrockner als besonders vorteilhaft herausgestellt. Die einzelnen Verfahrensparameter, wie aufgegebene Polymerisatmenge, Luftmenge, Menge des Agglomerierungshilfsmittels und Lufttemperatur sowie Dauer der Agglomerierungsbehandlung lassen sich durch einfache Versuche ermitteln.

Die Agglomerierung kann auch in einem Mischer, der mit einer Einrichtung zur Besprühung und mit rotierendem Mischwerkzeug, das mit Leitschaufeln, die die Wände des Mischers gründlich abstreifen, ausgerüstet ist, durchführen. Nachdem eine bestimmte Drehzahl des Rührwerks erreicht wird, werden beim Austreten der rotierenden Leitschaufeln des Mischwekzeuges aus der Gutmasse die einzelnen Körnchen herausgeschleudert, und es wird auf diese Weise ein Pulverfließbett gebildet. In diesem Stadium erfolgt die Aufsprühung des dispergierten oder gelösten Agglomerierungshilfsmittels mit einer Düse. Das Gerät kann bei Raumtemperatur oder bei vorhandenem Heiz- oder Kühlmantel bei höheren oder niedrigeren Temperaturen betrieben werden.

Als Polymerisate, die man auf diese Weise agglomerieren kann, sind sowohl die wasserlöslichen und wasserquellbaren Polymeren auf der Basis von Polysacchariden, wie Cellulose, Cellulosederivate wie carboxymethylcellulose, Alkyl- oder Hydroxyalkylcellulose, Stärke und Stärkederivate und Pflanzengummi (Xanthangummi, Alginsäure) und ihre Salze als auch die Polymeren oder Copolymeren auf der Basis von (Meth-)Acrylsäure oder (Meth-)Acrylsäurederivaten geeignet, wobei es sich hierbei in erster Linie um die Homo- oder Copolymere der Acryl-, Methacryl-, Acrylamindomethylpropansulfonsäure, der Salze dieser Säuren, des Acryl- oder Methacrylamids untereinander oder mit Vinylpyrrolidon und/oder Vinylacetat handelt. Die vorstehenden Polymeren können auch durch einen mindestens bifunktionellen Vernetzer vernetzt sein, damit sie in Wasser nur quellbar, aber nicht löslich sind. Alle diese Polymeren werden nach bekannten Verfahren hergestellt.

Als Agglomerierungshilfsmittel kann das Ausgangsmaterial selbst, d.h. wasserlöslichen oder wasserquellbaren Polymerisate, auf synthetischer oder natürlicher Grundlage (Polysaccharidbasis in gelöstem oder aufgequollenem Zustand) verwendet werden. Beispiele für solche wasserlösliche oder wasserquellbare Polymerisate auf natürlicher Basis sind Stärke, insbesondere Maisstärke, Cellulosederivate wie Carboxymethylcellulose, Alkyl- oder Hydroxyalkylcellulose, Stärke und Stärkederivate und Pflanzengummi (Xanthangummi, Alginsäure) und ihre Salze als auch auf synthetischer Basis die Polymeren oder Copolymeren auf der Basis von (Meth-)Acrylsäure oder (Meth)-Acrylsäurederivaten wie die Homo- oder Copolymere der Acryl-, Methacryl-, Acrylamindomethylpropansulfonsäure, der Salze dieser Säuren, des Acryl- oder Methacrylamids untereinander oder mit Vinylpyrrolidon und/oder Vinylacetat sowie Polyvinylalkohol. Die vorstehenden Polymeren können auch durch einen mindestens bifunktionellen Vernetzer vernetzt sein, damit sie in Wasser nur quellbar, aber nicht löslich sind.

Es können aber auch nieder- oder hochmolekulare Polymerisate, die als Emulsionspolymerisate in einer wäßrigen Dispersion in Form der durch Emulgator solubilisierten winzigen kugelförmigen Partikel vorhanden sind, verwendet werden, wobei beide Emulsionsformen "Öl-in-Wasser" (für wasserunlösliche Polymere) sowie "Wasser-in-Öl" (für wasserlösliche Polymere) möglich sind. Die Ölphase besteht bekanntlich meist aus mit Wasser nicht mischbaren organischen Lösungsmitteln wie aliphatische oder aromatische Kohlenwasserstoffe (z.B. Hexan, Toluol).

Beispiele für Polymerisate, die Öl-in-Wasser-Emulsionen bilden können, sind Polymerisate von Butadien, Styrol, Isopren, Chloropren, Acrylnitril, Vinylacetat, Vinyl- und Vinylidenchlorid, Alkylacrylate und -methacrylate und Copolymerisate von diesen Monomeren untereinander sowie auch mit Methylstyrol, Isobutylen oder Ethylen.

Als Beispiele für Polymerisate, die in Wasser-in-Öl-Emulsionen verwendet werden, können die schon o.a. wasserlöslichen oder wasserquellbaren Polymerisate bzw. Copolymerisate auf Basis von (Meth)-Acrylsäurederivaten, die unvernetzt oder auch vernetzt sein können.

Darüber hinaus kann man auch andere Substanzen, die zur Agglomerierung befähigt sind, verwenden. So können als Agglomerierungshilfsmittel auch solche Produkte verwendet werden, die gemäß DE-PS 31 28 100 zur Verbesserung der Flüssigkeitsaufnahmegeschwindigkeit den Polymerisaten zugesetzt werden.

Zur Ermittlung der Aufnahmegeschwindigkeit wird die Aufnahme von Modellurin nach dem Demand-Absorbency-Test Test (W.F. Schlauch, Vortrag Index 1978, Amsterdam) durchgeführt und die Aufnahmegeschwindigkeit nach 60 Sekunden sowie die Maximalaufnahme und Retention nach 20 bzw. 40 Minuten ermittelt. Das Meßgerät besteht aus einer Bürette, die mit der Modellurinlösung (2,0 % Harnstoff, 0,9 % NaCl, 0,1 % $MgSO_4$ und 0,06 % $CaCl_2$ aufgelöst in destilliertem Wasser) gefüllt ist, und einem Probetisch, der mit einer an die Meßbürette angeschlossenen Öffnung für den Modellurinlösungaustritt vorgesehen ist. Auf dem mit einem dünnen Vlies (10 X 13,5 cm) bedeckten Probetisch wird 0,5 g des erfindungsgemäßen Produkts vermischt mit 5 mg Aerosil 200 in Form einer kreisrunden Fläche von 4,5 cm Durchmesser, zentrisch über dem Flüssigkeitsaustritt, gleichmäßig aufgestreut. Durch Schließen des Schlauches und leichte Druckgebung wird der Kontakt der Modellurinlösung mit dem Pulverprodukt hergestellt, so daß die Modellurinlösung durch das erfindungsgemäße Produkt absorbiert werden kann. Die absorbierende Menge der Modellurinlösung wird nach 60 Sekunden und nach 20 - 30 Minuten der erste Maximalwert abgelesen. Anschließend wurde die Retention durch Belastung des gequollenen Geles mit einem Gewicht von 10 $g/cm^2$ ermittelt; die Zeit der Belastung betrug 5 Minuten. Die ermittelten 60-Sekunden-, Maximal- und Retentionswerte sind in den Beispielen tabellenweise zusammengefaßt.

3

Als weitere Methode zur Bestimmung der Flüssigkeitsaufnahmegeschwindigkeit während kurzer Zeit wurde ein Teebeuteltest durchgeführt, wobei die Flüssigkeitsaufnahme von 0,2 g Prüfsubstanz ohne Aerosilzusatz in einem Teebeutel nach 15 Sekunden gravimetrisch ermittelt und auf 1 g Produkt umgerechnet wurde.

Die Erfindung betrifft ferner Wegwerferzeugnisse für Hygieneartikel, wie z.B. Windeln oder Damenbinden, enthaltend als Absorptionsmittel für Körperflüssigkeiten, wie Wasser und Urin ein Polymerisat nach Anspruch 1.

Beispiele 1 bis 7:

Die Agglomerierung des pulverigen Polymerisats wurde in einem Wirbelschichtbettschichttrockner durchgeführt. Das Gerät besteht aus einem vertikalen konischen Metallzylinder, der am unteren Ende mit einem Siebboden abgeschlossen und einer Einrichtung zum Einsaugen von Luft versehen ist. Das zu behandelnde körnige Produkt wird in diesem Zylinder von einer regulierbaren Luftmenge durchströmt, bis die einzelnen Körnchen in Bewegung geraten, wodurch das Fließ- oder Wirbelbett gründlich durchmischt wird. In diesem Stadium erfolgt die Aufsprühung des gelösten Agglomerierungshilfsmittels mit Hilfe einer Düse. Die Menge des Hilfsstoffes, der Luftmenge sowie die Lufttemperatur können in bestimmten Grenzen variiert werden.

Für die Beispiele 1 bis 7 wurde das Gerät jeweils mit 1000 g feingemahlener pulvriger, teilweise vernetzter Polyacrylsäure, mit 90 Gew.-% Kornanteil unter 90 $\mu$ und einer Modellurinaufnahmegeschwindigkeit von 15 ml/g (DAT 60 sec-Wert) gefüllt und auf 110°C mit durchströmender Luftmenge erwärmt. Danach wurde das Polymerisat im Wirbelbett innerhalb von 5 - 15 Minuten durch Aufsprühen der wäßrigen Lösungen von niedermolekularen Polyacrylsäuren agglomeriert. Die Versuchsbedingungen und die Ergebnisse sind in der nachfolgenden Tabelle zusammengefaßt. Es wurde eine Ausbeute an den agglomerierten Partikeln von 89 - 95 Gew.-% der Kornfraktion 90 - 630 $\mu$m erhalten, wobei das agglomerierte Produkt eine Verbesserung der Anfangsabsorptionsgeschwindigkeit (DAT-60-Sec-Wert) von 15 bis auf 230 ml und mehr zeigte.

Tabelle 1: <u>Agglomerierung mit niedermolekularen Polyacrylsäuren</u>

| Beispiel | Zusatzstoff Agglomerierungshilfs- mittel | Gew.-% | Wasser- menge Gew.-% | DAT-Werte mit 0,5 % Aerosil | | | Kornverteilung (µm) | | |
|---|---|---|---|---|---|---|---|---|---|
| | | | | 60 sec. | Max | Ret | <90 | 90-630 | >630 |
| 1 | Polyacrylsäure $M_v$=90.000 g/mol | 0,57 | 9,4 | 21,3 | 46,1 | 24,4 | 6,5 | 93,5 | 0 |
| 2 | " | 0,29 | 9,7 | 21,6 | 44,1 | 25,0 | 6,5 | 93,5 | 0 |
| 3 | Polyacrylsäure $M_v$=45.000 g/mol | 1,14 | 10,0 | 19,0 | 46,2 | 26,1 | 10,0 | 88,8 | 1,2 |
| 4 | " | 0,57 | 10,0 | 23,8 | 47,6 | 25,0 | 9,5 | 90,5 | 0 |
| 5 | Polyacrylsäure $M_v$=14.000 g/mol | 0,25 | 19,7 | 20,4 | 48,0 | 25,5 | 9,2 | 90,8 | 0 |
| 6 | " | 0,5 | 19,5 | 20,4 | 48,4 | 25,8 | 8,1 | 91,9 | 0 |
| 7 | " | 1,0 | 19,0 | 20,4 | 43,7 | 24,7 | 5,1 | 94,9 | 0 |
| Vergleich Ausgangsstoff: | | – | – | 15,3 | 42,5 | 24,8 | 90 | 10 | 0 |

<u>Beispiele 8 bis 12:</u>

Im gleichen Gerät wurde das feingemahlene Polymerisat wie in Beispielen 1 bis 7 aber mit anderer Kornverteilung (88 Gew.-% Kornanteil unter 150 µm, s.Tabelle 2) bei 120 °C Lufttemperatur mit wäßriger Lösung von hochmolekularem Acrylsäure/Acrylamid-Copolymerisat ($M_v = 8.10^6$ g/mol) mit und ohne Natriumchloridzusatz agglomeriert. Es wurde eine Ausbeute an der Kornfraktion 150 - 850 µm von 53 bis 90 Gew.-% erhalten sowie eine Verbesserung der Anfangsabsorptionsgeschwindigkeit. Die Ergebnisse sind in der nachfolgenden Tabelle zusammengefaßt.

Tabelle 2: Agglomerierung mit hochmolekularem Acrylamid/Acrylsäure Copolymerisat (Praestol 2995) ohne und mit Zusatz von Natriumchlorid

| Beispiel | Zusatzstoff Agglomerierungshilfs-mittel | Gew.-% | Wasser-menge Gew.-% | DAT-Werte mit 0,5 % Aerosil 60 sec | Max | Ret | Kornverteilung (µm) <45 | 45-150 | 150-850 | >850 |
|---|---|---|---|---|---|---|---|---|---|---|
| 8 | Praestol 2995 | 0,01 | 10,0 | 23,0 | 50,8 | 25,7 | 1,0 | 6,2 | 90,3 | 2,5 |
| 9 | " | 0,025 | 25,0 | 23,0 | 47,2 | 24,6 | 0,5 | 35,1 | 62,2 | 0,5 |
| 10 | " | 0,05 | 50,0 | 22,6 | - | - | 2,0 | 29,5 | 67,3 | 1,2 |
| 11 | Praestol 2995 NaCl | 0,05 0,25 | 25,0 | 25,2 | 50,7 | 25,1 | 3,2 | 34,3 | 53,5 | 8,9 |
| 12 | Praestol 2995 NaCl | 0,05 0,50 | 25,0 | 16,8 | 47,8 | 25,8 | 1,3 | 41,3 | 57,1 | 0,3 |
| Vergleich Ausgangsstoff: | | - | - | 15,8 | 48,6 | 24,7 | 8,0 | 80,0 | 12,0 | 0 |

Beispiele 13 bis 15:

Ein feingemahlenes Acrylsäurecopolymerisat mit einem sehr niedrigen Vernetzungsgrad und 100 Gew.-% Kornanteil unter 90 $\mu$m (Modellurinaufnahmegeschwindigkeit -DAT 60 sec-Wert von 2,2 ml/g) wurde bei 120°C mit wäßriger Lösung von hochmolekularem Acrylsäure/Acrylamid-Copolymerisat ($M_v = 8.10^6$ g/mol) agglomeriert und anschließend mit wäßriger Lösung von Aluminiumsulfat bei gleicher Temperatur besprüht. Nach dieser Behandlung stieg bei diesem Produkt die Anfangsabsorptionsgeschwindigkeit von 2,2 ml/min bis auf 12-14 ml/min, wobei eine Ausbeute an der Kornfraktion 90 - 630 $\mu$m von 72 - 85 Gew.-% erzielt wurde. Die Versuchsbedingungen und die Ergebnisse sind in Tabelle 3 aufgeführt.

Tabelle 3: Agglomerierung mit Praestol 2995 als Agglomerierungsmittel und mit nachfolgender Behandlung des Agglomerates mit Aluminiumsulfatlösung

| Beispiel | Zusatzstoff Agglomerierungs- hilfsmittel | Gew.-% | Wasser- menge Gew.-% | DAT-Werte mit 0,5 % Aerosil | | | Kornverteilung (μm) | | |
|---|---|---|---|---|---|---|---|---|---|
| | | | | 60 sec | Max | Ret | < 90 | 90- 630 | > 630 |
| 13 | Praestol 2995 | 0,03 | 20,0 | 11,9 | 48,0 | 35,7 | 14,7 | 84,6 | 0,7 |
| | Alusulfat | 0,58 | 10,0 | | | | | | |
| 14 | Praestol 2995 | 0,04 | 20,0 | 14,5 | 46,5 | 33,4 | 16,6 | 75,4 | 8,0 |
| | Alusulfat | 0,58 | 10,0 | | | | | | |
| 15 | Praestol 2995 | 0,05 | 20,0 | 11,6 | 46,8 | 35,0 | 14,6 | 72,2 | 13,2 |
| | Alusulfat | 0,58 | 10,0 | | | | | | |
| Vergleich Ausgangsstoff | - | - | - | 2,2 | 36,2 | - | 100 | 0 | 0 |

Beispiele 16 und 17:

Ein feingemahlenes, teilvernetztes Acrylsäurecopolymerisat mit 96 Gew.-% Kornanteil unter 90 $\mu$ und Modellurinaufnahmegeschwindigkeit von 18 ml/g (15 sec, Teebeuteltest) wurde bei 50 °C mit wäßriger Lösung von niedermolekularer Polyacrylsäure ($M_v = 6.000$), in der gleichzeitig ein sehr fein gemahlenes, schwach vernetztes Polymerisat aufgequollen wurde, im gleichen Gerät wie in den vorherigen Beispiele agglomeriert. Die Lösung des Agglomerierungshilfsmittels wurde mit einer Schlauchpumpe dosiert und unter Druck versprüht.

Es wurde eine Ausbeute an der Kornfraktion 90 - 300 $\mu$m von 60 bis 65 Gew.-% und an der Kornfraktion von 300 - 800 $\mu$m von ca. 24 Gew.-% erhalten. Die Ergebnisse und Versuchsbedingungen sind in der nachfolgenden Tabelle 4 zusammengefaßt.

Tabelle 4 : Agglomerierung mit in Polyelektrolytlösung aufgequollenem, leicht vernetztem Polymerisat

| Beispiel | Zusatzstoff Agglomerierungs- hilfsmittel | Gew.-% | Wasser- menge Gew.-% | Kornverteilung | | | | Teebeuteltest 15 Sekunden (ml/g) |
|---|---|---|---|---|---|---|---|---|
| | | | | <90 | 90– 300 | 300– 800 | >800 | |
| 16 | Polyacrylsäure $M_V$ = 6.000 Polyacrylsäure vernetzt | 0,1 0,1 | 10 | 4,1 | 61,6 | 23,6 | 10,3 | 31 |
| 17 | Polyacrylsäure $M_V$ = 6.000 Polyacrylsäure vernetzt | 0,05 0,1 | 10 | 4,7 | 65,0 | 24,3 | 6,0 | 33 |
| Vergleich Ausgangsstoff | – | – | 96 | 4 | 0 | 0 | 18 |

Die agglomerierten Produkte zeigen deutlich höhere Flüssigkeitsaufnahmegeschwindigkeit, geprüft in einem Teebeuteltest (15 Sekunden Tauchzeit) als das nicht-agglomerierte Ausgangsprodukt.

Beispiele 18 und 19:

Ein feingemahlenes, hochmolekulares, wasserlösliches Polyacrylamid mit 92 Gew.-% Kornanteil unter 90 $\mu$m und Molekulargewicht von $4.10^6$ wurde bei 65 °C mit wäßriger Lösung von Polyacrylsäure im Wirbelschichttrockner agglomeriert.

Es wurde eine Ausbeute von 80 - 85 Gew.-% der Kornfraktion 90 - 300 μm und 6 - 20 Gew.-% der Kornfraktion 300 - 800 μm erhalten. Die Versuchsbedingungen und Ergebnisse sind in der Tabelle 5 zusammengefaßt.

Tabelle 5: Agglomerierung von hochmolekularem, wasserlöslichem Polymerisat

| Beispiel | Zusatzstoff Agglomerierungs-hilfsmittel | Gew.-% | Wasser-menge Gew.-% | Kornverteilung | | |
|---|---|---|---|---|---|---|
| | | | | <90 | 90-300 | 300-800 |
| 18 | Polyacrylsäure $M_V$=8.000 | 0,03 | 10,0 | 8,6 | 85,0 | 6,3 |
| 19 | Polyacrylsäure $M_V$ = 200.000 | 0,11 | 15,0 | 0,6 | 79,5 | 19,9 |
| Ausgangsprodukt | | – | – | 92 | 8 | – |

Bei der Herstellung von 0,1 %igen Lösungen zeigte sich, daß die agglomerierten Produkte (Beispiel 18 und 19) sich sehr schnell, innerhalb von 20 - 30 Sekunden, in Wasser vollständig lösen. Im Gegensatz dazu löst sich das Ausgangsprodukt deutlich langsamer, bei der Herstellung der Lösung unter gleichen Bedingungen bilden sich Klumpen, und es werden 20 - 30 Minuten gebraucht, bis eine homogene Lösung entsteht.

Beispiele 20 bis 22:

Ein feingemahlenes, teilvernetztes Acrylsäurecopolymerisat mit 92 Gew.-% Kornanteil unter 90 $\mu$ und einer Modellurinaufnahmegeschwindigkeit von 18 ml/g (Teebeuteltest, 15 sec) bei 90°C mit wäßriger Lösung eines Agglomerierungshilfsmittels (Stärke, Polyvinylalkohol) im gleichen Gerät wie in den vorherigen Beispielen agglomeriert. Die Lösung wurde mit einer Schlauchpumpe dosiert und unter Stickstoffdruck versprüht. Es wurde eine Ausbeute an der Kornfraktion 90 - 300 $\mu$m von 55 - 65 Gew.-% und an der Kornfraktion von 300 - 800 $\mu$m von 32 - 42 Gew.-% erhalten. Die Ergebnisse und Versuchsbedingungen sind in der Tabelle 6 zusammengefaßt.

Tabelle 6: <u>Agglomerierung mit hydroxygruppenhaltigen Polymeren</u>

| Beispiel | Zusatzstoff Agglomerierungs-hilfsmittel | Gew.-% | Wasser-menge Gew.-% | Kornverteilung | | | | Teebeuteltest (ml/g) |
|---|---|---|---|---|---|---|---|---|
| | | | | <90 | 90-300 | 300-800 | >800 | |
| 20 | Maisstärke | 0,075 | 15 | 0 | 66,3 | 32 | 1,1 | 36,1 |
| 21 | Maisstärke | 0,3 | 15 | 0 | 64,9 | 33 | 1,6 | 34,3 |
| 22 | Polyvinyl-alkohol | 0,4 | 15 | 0 | 56,1 | 42 | 1,4 | 33,6 |
| Ausgangsprodukt | - | - | | 92 | 8 | 0 | 0 | 18 |

Beispiele 23 bis 26:

Ein feingemahlenes Acrylsäurepolymerisat wie in den Beispielen 20 - 22 wurde bei 80 °C im Wirbelbetttrockner mit Lösung einer wasserlöslichen Verbindung (Komponente B) gemäß DE-PS 31 28 100

13

besprüht. Die Lösung wurde mit einer Schlauchpumpe dosiert und mit Druckluft versprüht. Die Ergebnisse und Versuchsbedingungen sind in der Tabelle 7 zusammengefaßt.

Tabelle 7: Agglomerierung mit wasserlöslichen Verbindungen gemäß DE-PS 31 28 100 (Komponente B)

| Beispiel | Zusatzstoff Agglomerierungs-hilfsmittel | Gew.-% | Wasser-menge Gew.-% | Kornverteilung | | | | Teebeuteltest (ml/g) |
|---|---|---|---|---|---|---|---|---|
| | | | | <90 | 90-300 | 300-800 | >800 | |
| 23 | Aluminiumsulfat | 0,4 | 15 | 0 | 63,8 | 27,4 | 7,8 | 34,6 |
| 24 | Harnstoff | 0,4 | 15 | 0 | 67,1 | 32,2 | 1,1 | 35,2 |
| 25 | Citronensäure | 0,4 | 15 | 0 | 78,1 | 21,2 | 0,2 | 35,7 |
| 26 | " | 1,0 | 15 | 0 | 74,8 | 24,5 | 0 | 34,8 |
| Ausgangsprodukt | | – | – | 92 | 8 | 0 | 0 | 18 |

Beispiele 27 - 33:

Ein feingemahlenes, teilvernetztes Acrylsäurecopolymerisat mit 91,5 Gew.-% Kornanteil unter 90 $\mu$m und Modellurinaufnahmegeschwindigkeit von 18 ml/g (15 sec Teebeuteltest) wurde bei 40°C mit wäßriger Dispersion von Polyacrylsäureester oder Polyvinylacetat im gleichen Gerät wie in den vorherigen Beispielen agglomeriert. Die Dispersion des Agglomerierungshilfsmittels, evtl. mit Wasser verdünnt, wurde mit einer Schlauchpumpe dosiert und unter Druck versprüht. Es wurde eine Ausbeute an der Kornfraktion 90 - 300 $\mu$m von 50 bis 88 Gew.-% und an der Kornfraktion von 300 - 800 $\mu$m von 10 bis 50 Gew.-% erhalten. Die Ergebnisse und Versuchsbedingungen sind in der nachfolgenden Tabelle 8 zusammengefaßt.

Beispiele 34-38:

Die Agglomerierung des pulverigen Polymerisats wurde in einem Mischer mit rotierendem Mischwerkzeug durchgeführt. Das Gerät besteht aus einem vertikal oder horizontal gelegenen Metallzylinder (Volumen

Tabelle 8: Agglomerierung mit wäßrigen Kunststoffdispersionen

| Bei-spiel | Zusatzstoff Agglomerierungs-hilfsmittel | Gew% | Wasser-menge Gew% | Kornverteilung | | | | DAT-Werte (ml/g) | | | Teeb.test 15sec-Wert (ml/g) |
|---|---|---|---|---|---|---|---|---|---|---|---|
| | | | | <90 | 90-300 | 300-800 | >800 | 60 sec | Max | Ret | |
| 27 | Polyacrylsäureethylester 1) 45%ige Dispersion in Wasser | 4,5 | - | 0 | 57,6 | 40,5 | 1,6 | 29,1 | 49,4 | 26,5 | 32,0 |
| 28 | " " | 4,5 | 5,5 | 0 | 55,2 | 44,0 | 0,8 | 26,0 | 50,4 | 27,2 | 30,5 |
| 29 | " " | 2,2 | 2,8 | 0 | 70,1 | 29,5 | 0 | 29,1 | 50,4 | 27,0 | 31,5 |
| 30 | Acrylsäureestercopolymer. 2) 55%ige Dispersion in Wasser | 1,7 | 3,3 | 0 | 60,3 | 38,8 | 0,6 | 24,5 | 48,6 | 26,4 | - |
| 31 | " " | 3,3 | 6,7 | 0 | 48,3 | 50,0 | 1,7 | 24,5 | 48,4 | 26,8 | 31,0 |
| 32 | Vinylacetat/Ethylen-Copolymerisat 3) 60%ige Dispersion in Wasser | 2,5 | 2,5 | 0 | 88,6 | 10,3 | 0,7 | 25,6 | 48,4 | 25,9 | - |
| 33 | " " | 5,0 | 5,0 | 0 | 84,7 | 13,4 | 1,6 | 26,7 | 51,2 | 26,3 | - |
| Vergleich Ausgangsprodukt | | - | - | 91,5 | 8,5 | 0 | 0 | 15,8 | 48,6 | 26,2 | 18 |

1) Sarpifan WRG [R]

2) Acrylsäuremethylester/Acrylsäurebutylester-Copolymerisat (Estekoll HL 40 [R])

3) Estekoll 60 [R]

6000 ml), dessen Mischwerkzeug mit Leitschaufeln, die die Wände des Mischers gründlich abstreifen, und einer Einrichtung zur Besprühung ausgerüstet ist. Nachdem eine bestimmte Drehzahl (300 Upm) des Rührwerks erreicht wurde, bildet sich beim Austreten der rotierenden Leitschaufeln des Mischwerkzeuges aus den herausgeschleuderten einzelnen Körnchen der Gutmasse ein Pulverfließbett auf der ganzen Länge des Metallzylinders. In diesem Stadium erfolgte die Aufsprühung des dispergierten oder gelösten Agglomerierungshilfsmittels mit einer Düse. Das Gerät wurde bei Raumtemperatur betrieben.

Für die Beispiele 34 bis 38 wurde das Gerät jeweils mit 1000 g feingemahlener pulvriger, teilweise vernetzter Polyacrylsäure, die 88 Gew.-% Kornanteil unter 90 $\mu$m und ein Modellurinaufnahmevermögen von 16,5 ml/g (15 sec Teebeuteltest) hatte, gefüllt und unter Rührung bei Normaltemperatur innerhalb von 5 - 15 Minuten durch Aufsprühen der wäßrigen Lösungen und/oder Dispersionen agglomeriert. Die Versuchsbedingungen und die Ergebnisse sind in der nachfolgenden Tabelle 9 zusammengefaßt. Es wurde eine gute Ausbeute an den agglomerierten Partikeln (84 - 99 Gew.-% der Kornfraktion 90 - 800 $\mu$m) erhalten, wobei das agglomerierte Produkt eine Verbesserung der Anfangsabsorptionsgeschwindigkeit (15 sec Teebeuteltest) von 16 (Ausgangsprodukt) auf 32 bis 45 ml/g zeigte.

Tabelle 9: Agglomerierung mit in Polyelektrolytlösung aufgequollenem, leicht vernetztem Polymerisat in einem Draismischer

| Bei-spiel | Zusatzstoff Agglomerierungs-hilfsmittel | Gew% | Wasser-menge Gew% | < 90 | 90-300 | 300-800 | > 800 | Teeb.test 15 sec-Wert (ml/g) |
|---|---|---|---|---|---|---|---|---|
| 34 | a) Polyacrylsäure vernetzt<br>b) Polyacrylsäure $M_v$ 8000 | 0,25<br>0,15 | 5,0 | 0 | 87,1 | 12,0 | 2,7 | 45,0 |
| 35 | a) Polyacrylsäure vernetzt<br>b) Polyacrylsäure $M_v$ 6000 | 0,50<br>0,30 | 10,0 | 0 | 55,3 | 28,6 | 19,0 | 41,0 |
| 36 | a) Polyacrylsäure vernetzt<br>b) Polyacrylsäure $M_v$ 8000<br>c) Polyacrylsäureester 55%ig in wäßriger Dispersion 1) | 0,48<br>0,30<br><br>0,25 | 4,75 | 0 | 57,2 | 32,4 | 11,9 | 32,0 |
| 37 | a) Acrylamidopolymerisat 2) 42 %ige Dispersion in Isohexa-dekan | 0,05 | 10,0 | 0 | 25,4 | 43,5 | 30,4 | 42,5 |
| 38 | a) Acrylamidopolymerisat 2) 42 %ige Dispersion in Isohexa-dekan<br>b) Isohexadekan | 4,0<br><br><br>6,0 | 1,2<br><br><br>– | 0 | 0 | 38,5 | 61,5 | 49,1 |
| Vergleich Ausgangsprodukt | | – | – | 88,0 | 7,5 | 4,5 | 0 | 16,5 |

1) Estekoll HL 40

2) Acrylamid/N-(Dimethylaminopropyl)acrylamid-40/60-Copolymerisat

EP 0 318 989 B1

**Patentansprüche**
**Patentansprüche für folgende Vertragsstaaten : AT, BE, CH, DE, FR, GB, GR, IT, LI, LU, NL, SE**

1. Wasserquellbare natürliche oder synthetische Polymerisate mit hohem Absorbierungsvermögen für Wasser und wässrige Flüssigkeiten, erhältlich durch Agglomerierung eines feinteiligen, pulverförmigen Ausgangsmaterials einer Korngröße von kleiner 0,2 mm, bevorzugt kleiner 0,1 mm und insbesondere kleiner 0,05 mm mittels einer Lösung oder Dispersion eines Agglomerierungshilfsmittels in Wasser, einem mit Wasser mischbaren oder nicht mischbaren organischen Lösungsmittel oder einem Gemisch aus Wasser und einem solchen organischen Lösungsmittel und gegebenenfalls weiteren, noch agglomerierend wirkenden Zusätzen.

2. Verfahren zur Herstellung von wasserquellbaren natürlichen oder synthetischen Polymerisaten mit hohem Absorptionsvermögen für Wasser und wässrige Flüssigkeiten, dadurch gekennzeichnet, daß man ein feinteiliges, pulverförmiges Ausgangsmaterial des Polymerisats einer Korngröße von kleiner 0,2 mm mittels einer Lösung oder Dispersion des Agglomerierungshilfsmittels in Wasser, einem mit Wasser mischbaren oder nicht mischbaren organischen Lösungsmittel oder einem Gemisch aus Wasser und einem mit Wasser mischbaren oder nicht mischbaren organischen Lösungsmittel in der Wirbelschicht agglomeriert.

3. Verfahren nach Anspruch 2, dadurch gekennzeichnet, daß man ein Ausgangsmaterial einer Korngröße von kleiner 0,1, bevorzugt kleiner 0,05 mm, einsetzt.

4. Verfahren nach einem der Ansprüche 2 bis 3, dadurch gekennzeichnet, daß die Agglomerierung durch Aufsprühen der wässrigen Lösung oder Mischung des Agglomerierungshilfsmittels vorgenommen wird.

5. Verfahren nach einem der Ansprüche 2 bis 4, dadurch gekennzeichnet, daß man als Ausgangsmaterial natürliche Polymerisate auf der Basis von Polysacchariden oder synthetische Polymerisate auf der Basis von (Meth-) Acrylsäurederivaten einsetzt, die gegebenenfalls schwach vernetzt sind.

6. Verfahren nach einem der Ansprüche 2 bis 4, dadurch gekennzeichnet, daß man als Agglomerierungshilfsmittel das zu agglomerierende Polymerisat selbst oder niedrigmolekulare Polymere auf der Basis von (Meth-)Acrylsäure einsetzt.

7. Verfahren nach Anspruch 1, dadurch gekennzeichnet, daß die Wirbelschicht mit erwärmter Luft betrieben wird.

8. Verwendung der Polymerisate nach Ansprüchen 1 bis 7 als Absorptionsmittel für Wasser und wässrige Flüssigkeiten, insbesondere Körperflüssigkeiten.

9. Wegwerferzeugnisse für Hygieneartikel, wie z. B. Windeln oder Damenbinden, enthaltend als Absorptionsmittel für Körperflüssigkeiten, wie Wasser und Urin, ein Polymerisat nach Anspruch 1 bis 7.

**Patentansprüche für folgenden Vertragsstaat : ES**

1. Verfahren zur Herstellung von Wasserquellbaren natürlichen oder synthetischen Polymerisaten mit hohem Absorptionsvermögen für Wasser und wässrige Flüssigkeiten, dadurch gekennzeichnet, daß man ein feinteiliges, pulverförmiges Ausgangsmaterial des Polymerisats einer Korngröße von kleiner 0,2 mm mittels einer Lösung oder Dispersion des Agglomerierungshilfsmittels in Wasser, einem mit Wasser mischbaren oder nicht mischbaren organischen Lösungsmittel oder einem Gemisch aus Wasser und einem mit Wasser mischbaren oder nicht mischbaren organischen Lösungsmittel in der Wirbelschicht agglomeriert.

2. Verfahren nach Anspruch 1, dadurch gekennzeichnet, daß man ein Ausgangsmaterial einer Korngröße von kleiner 0,1, bevorzugt kleiner 0,05 mm einsetzt.

3. Verfahren nach einem der Ansprüche 1 bis 2, dadurch gekennzeichnet, daß die Agglomerierung durch Aufsprühen der wässrigen Lösung oder Mischung des Agglomerierungshilfsmittels vorgenommen wird.

EP 0 318 989 B1

4. Verfahren nach einem der Ansprüche 1 bis 3, dadurch gekennzeichnet, daß man als Ausgangsmaterial natürliche Polymerisate auf der Basis von Polysacchariden oder synthetische Polymerisate auf der Basis von (Meth-) Acrylsäurederivaten einsetzt, die gegebenenfalls schwach vernetzt sind.

5. Verfahren nach einem der Ansprüche 2 bis 4, dadurch gekennzeichnet, daß man als Agglomerierungshilfsmittel das zu agglomerierende Polymerisat selbst oder niedrigmolekulare Polymere auf der Basis von (Meth-)Acrylsäure einsetzt.

6. Verfahren nach Anspruch 1, dadurch gekennzeichnet, daß die Wirbelschicht mit erwärmter Luft betrieben wird.

7. Wegwerferzeugnisse für Hygieneartikel, wie z. B. Windeln oder Damenbinden, enthaltend als Absorptionsmittel für Körperflüssigkeiten, wie Wasser und Urin, ein Polymerisat nach Anspruch 1 bis 6.

## Claims
### Claims for the following Contracting States : AT, BE, CH, DE, FR, GB, GR, IT, LI, LU, NL, SE

1. Water-swellable natural or synthetic polymers having a high absorption capacity for water and aqueous liquids, obtainable by agglomerating a finely divided, powdery starting material having a particle size of smaller than 0.2 mm, preferably smaller than 0.1 mm and in particular smaller than 0.05 mm, by means of a solution or dispersion of an agglomeration auxiliary agent in water, an organic solvent which is miscible or immiscible with water, or a mixture of water and such an organic solvent, and, optionally, additional additives having an agglomerating action.

2. A process for the manufacture of water-swellable natural or synthetic polymers having a high absorption capacity for water and aqueous liquids, characterized in that a finely divided, powdery starting material of the polymer having a particle size of smaller than 0.2 mm is agglomerated in the fluidized bed by means of a solution or dispersion of the agglomeration aid in water, an organic solvent which is miscible or immiscible with water, or a mixture of water and an organic solvent which is miscible or immiscible with water.

3. The process according to claim 2 characterized in that a starting material having a grain size of smaller than 0.1, preferably of smaller than 0.05 mm is used.

4. The process according to any one of claims 2 to 3 characterized in that the agglomeration is carried out by spraying the aqueous solution or the mixture of the agglomeration aid.

5. The process according to any one of claims 2 to 4 characterized in that natural polymers based on polysaccharides or synthetic polymers based on (meth-)acrylic acid derivatives, which are optionally slightly cross-linked, are used as starting material.

6. The process according to any one of claims 2 to 4 characterized in that the polymer to be agglomerated itself or low-molecular polymers based on (meth-)acrylic acid are used as agglomeration auxiliary agent.

7. The process according to claim 1 characterized in that the fluidized bed is powered by heated air.

8. The use of the polymers according to claims 1 to 7 as absorbents for water and aqueous liquids, in particular body fluids.

9. Disposable articles for hygienic products, such as diapers or sanitary napkins, comprising as absorbent for body fluids, such as water and urine, a polymer according to claims 1 to 7.

### Claims for the following Contracting State : ES

1. A process for the manufacture of water-swellable natural or synthetic polymers having a high absorption capacity for water and aqueous liquids, characterized in that a finely divided, powdery starting material of the polymer having a particle size of smaller than 0.2 mm is agglomerated in the fluidized bed by

20

EP 0 318 989 B1

means of a solution or dispersion of the agglomeration aid in water, an organic solvent which is miscible or immiscible with water, or a mixture of water and an organic solvent which is miscible or immiscible with water.

2. The process according to claim 1 characterized in that a starting material having a grain size of smaller than 0.1, preferably of smaller than 0.05 mm is used.

3. The process according to any one of claims 1 to 2 characterized in that the agglomeration is carried out by spraying the aqueous solution or the mixture of the agglomeration aid.

4. The process according to any one of claims 1 to 3 characterized in that natural polymers based on polysaccharides or synthetic polymers based on (meth-)acrylic acid derivatives, which are optionally slightly cross-linked, are used as starting material.

5. The process according to any one of claims 2 to 4 characterized in that the polymer to be agglomerated itself or low-molecular polymers based on (meth-)acrylic acid are used as agglomeration auxiliary agent.

6. The process according to claim 1 characterized in that the fluidized bed is powered by heated air.

7. Disposable articles for hygienic products, such as diapers or sanitary napkins, comprising as absorbent for body fluids, such as water and urine, a polymer according to claims 1 to 6.

**Revendications**

**Revendications pour les Etats contractants suivants : AT, BE, CH, DE, FR, GB, GR, IT, LI, LU, NL, SE**

1. Polymères naturels ou synthétiques, gonflables à l'eau, à haut pouvoir d'absorption d'eau et de liquides aqueux, que l'on peut obtenir par l'agglomération d'une matière de départ pulvérulente, finement divisée, d'un calibre des grains inférieur à 0,2 mm, de préférence inférieur à 0,1 mm et, plus particulièrement encore, inférieur à 0,05 mm, à l'aide d'une solution ou d'une dispersion d'un adjuvant d'agglomération dans l' eau, d'un solvant organique, miscible à l'eau ou non miscible à l'eau, ou d'un mélange d'eau et d'un solvant organique du genre précité et, éventuellement, d'autres additifs à activité agglomérante.

2. Procédé de préparation de polymères naturels ou synthétiques, gonflables à l'eau, à haut pouvoir d'absorption d'eau et de liquides aqueux, caractérisé en ce que l'on agglomère une matière de départ du polymère pulvérulente et finement divisée, d'un calibre des grains inférieur à 0,2 mm, à l'aide d'une solution ou d'une dispersion de l'adjuvant d'agglomération dans l'eau, d'un solvant organique, miscible à l'eau ou non miscible à l'eau, ou d'un mélange d'eau et d'un solvant organique, miscible à l'eau ou non miscible à l'eau, en couche turbulente ou fluidisée.

3. Procédé suivant la revendication 2, caractérisé en ce que l'on utilise une matière de départ d'un calibre des grains inférieur à 0,1 mm, de préférence inférieur à 0,05 mm.

4. Procédé suivant l'une quelconque des revendications 2 et 3, caractérisé en ce que l'on entreprend l' agglomération par la pulvérisation du mélange ou de la solution dans l'eau de l'adjuvant d'agglomération.

5. Procédé suivant l'une quelconque des revendications 2 à 4, caractérisé en ce que l'on utilise, à titre de matière de départ, des polymères naturels à base de polysaccharides, ou des polymères synthétiques à base de dérivés de l'acide (méth)acrylique, qui sont éventuellement faiblement réticulés.

6. Procédé suivant l'une quelconque des revendications 2 à 4, caractérisé, en ce qu'à titre d'adjuvant d'agglomération, on utilise le polymère à agglomérer lui-même, ou des polymères de faibles poids moléculaires à base d'acide (méth)acrylique.

7. Procédé suivant la revendication 1, caractérisé en ce que l'on réalise la couche turbulente ou fluidisée à l'aide d'air chauffé.

**8.** Utilisation des polymères suivant l'une quelconque des revendications 1 à 7, à titre d'agents d'absorption d'eau ou de liquides aqueux, plus particulièrement de liquides corporels.

**9.** Objets à jeter pour des articles d'hygiène, comme, par exemple des couches ou des serviettes hygiéniques, contenant, à titre d'agent d'absorption de liquides corporels, comme l'eau et l'urine, un polymère suivant l'une quelconque des revendications 1 à 7.

**Revendications pour l'Etat contractant suivant : ES**

**1.** Procédé de préparation de polymères naturels ou synthétiques, gonflables à l'eau, à haut pouvoir d'absorption d'eau et de liquides aqueux, caractérisé en ce que l'on agglomère une matière de départ du polymère pulvérulente et finement divisée, d'un calibre des grains inférieur à 0,2 mm, à l'aide d'une solution ou d'une dispersion de l'adjuvant d'agglomération dans l'eau, d'un solvant organique, miscible à l'eau ou non miscible à l'eau, ou d'un mélange d'eau et d'un solvant organique, miscible à l'eau ou non miscible à l'eau, en couche turbulente ou fluidisée.

**2.** Procédé suivant la revendication 1, caractérisé en ce que l'on utilise une matière de départ d'un calibre des grains inférieur à 0,1 mm, de préférence inférieur à 0,05 mm.

**3.** Procédé suivant l'une quelconque des revendications 1 et 2, caractérisé en ce que l'on entreprend l'agglomération par la pulvérisation du mélange ou de la solution dans l'eau de l'adjuvant d'agglomération.

**4.** Procédé suivant l'une quelconque des revendications 1 à 3, caractérisé en ce que l'on utilise, à titre de matière de départ, des polymères naturels à base de polysaccharides, ou des polymères synthétiques à base de dérivés de l'acide (méth)acrylique, qui sont éventuellement faiblement réticulés.

**5.** Procédé suivant l'une quelconque des revendications 2 à 4, caractérisé, en ce qu'à titre d'adjuvant d'agglomération, on utilise le polymère à agglomérer lui-même, ou des polymères de faibles poids moléculaires à base d'acide (méth)acrylique.

**6.** Procédé suivant la revendication 1, caractérisé en ce que l'on réalise la couche turbulente ou fluidisée à l'aide d'air chauffé.

**7.** Objets à jeter pour des articles d'hygiène, comme, par exemple des couches ou des serviettes hygiéniques, contenant, à titre d'agent d'absorption de liquides corporels, comme l'eau et l'urine, un polymère suivant l'une quelconque des revendications 1 à 6.